# EUROPÄISCHE PATENTANMELDUNG

(11) **EP 4 462 439 A1**
(43) Veröffentlichungstag der Anmeldung: **13.11.2024**
(21) Anmeldenummer: 23172820.5
(22) Anmeldetag: 11.05.2023
(51) Int. Cl.: G16B 50/50

(54) **VERFAHREN ZUM OPTIMIERTEN KOMPRIMIEREN VON QUALITÄTSMARKERN SOWIE COMPUTEREINRICHTUNG MIT EINER KOMPRIMIERUNGSEINHEIT**

(71) Anmelder: Gottfried Wilhelm Leibniz Universität Hannover, 30167 Hannover (DE)
(72) Erfinder: Ostermann, Jörn, 30655 Hannover (DE); Voges, Jan, 30459 Hannover (DE)
(74) Vertreter: Weidner Stern Jeschke

(57) **Zusammenfassung**

Die Erfindung betrifft ein Verfahren zum optimierten Komprimieren von Qualitätsmarkern einer aus einem Genom bestimmten Genomsequenz mittels einer Komprimierungseinheit, wobei die Genomsequenz an jeweiligen Genompositionen jeweilige Genominformationen aufweist und jeweilige Qualitätsmarker der jeweiligen Genomposition zugeordnet sind und einen Qualitätswert einer Genominformation an der jeweiligen Genomposition in der Genomsequenz aufweisen. Des Weiteren betrifft die Erfindung eine Computereinrichtung mit einer Komprimierungseinheit.

## Beschreibung

Die Erfindung betrifft ein Verfahren zum optimierten Komprimieren von Qualitätsmarkern einer aus einem Genom bestimmten Genomsequenz mittels einer Komprimierungseinheit, wobei die Genomsequenz an jeweiligen Genompositionen jeweilige Genominformationen aufweist und die jeweiligen Qualitätsmarker der jeweiligen Genomposition zugeordnet sind und einen Qualitätswert einer Genominformation an der jeweiligen Genomposition in der Genomsequenz aufweisen. Weiterhin betrifft die Erfindung eine Computereinrichtung mit einer Komprimierungseinheit.

Es sind Verfahren bekannt, um Qualitätsmarker einer aus einem Genom bestimmten Genomsequenz zu komprimieren. Allerdings besteht bei diesen Verfahren ein direkter Zusammenhang zwischen Komprimierungsgrad und Verlust von Information bei der Weiterverarbeitung der Genomsequenz.

Beispielsweise werden sogenannte heterogene Konsens-Algorithmen genutzt, um Konsens-Genominformationen jeder Genomposition zu erzeugen. Weiterhin sind Komprimierungsverfahren bekannt, bei denen Qualitätsinformationen, also beispielsweise Qualitätsmarker, durch eine Glättungsfunktion geschachtelt oder geglättet werden. Insbesondere bei der Weiterverarbeitung entsprechender Qualitätsmarker wird dann jedoch häufig ein Qualitätsverlust deutlich. Weiterhin sind Verfahren bekannt, bei welchen Qualitätsmarker unter Nutzung eines Markow-Prozesses erster Ordnung modelliert werden.

Die US 10,938,414 B2 beschreibt ein Verfahren zum Kodieren und Dekodieren von Qualitätsmarkern, wobei hier eine Verschlüsselungstabelle zur Anwendung kommt.

Die US 10,691,775 B2 beschreibt ein sogenanntes Bioinformatisches System, eine Apparatur sowie Verfahren zum Analysieren von genetischen Informationen.

Die Dokumente CN 111640467 A sowie CN 10539145 A betreffen ebenfalls Datenkompression.

Die WO 2020/191390 A2 offenbart die Allokation von Qualitätsmarkern zu Genomsequenzen.

Die WO 20157180203 A1 beschreibt ein verlustfreies Kompressionssystem für Qualitätsmarker und ein entsprechendes Verfahren.

Die WO 2018/068845 A1 sowie die WO 2019/012153 A1 offenbaren jeweils Verfahren zum Kodieren und Dekodieren von Qualitätswerten einer Datenstruktur.

Aufgabe der Erfindung ist es, den Stand der Technik zu verbessern.

Gelöst wird die Aufgabe durch ein Verfahren zum optimierten Komprimieren von Qualitätsmarkern einer aus einem Genom bestimmten Genomsequenz mittels einer Komprimierungseinheit, wobei die Genomsequenz an jeweiligen Genompositionen jeweilige Genominformationen aufweist und jeweilige Qualitätsmarker der jeweiligen Genomposition zugeordnet sind und einen Qualitätswert einer Genominformation an der jeweiligen Genomposition in der Genomsequenz aufweisen, mit folgenden Schritten:
- Einlesen des jeweiligen Qualitätsmarkers zur jeweiligen Genomposition in die Komprimierungseinheit, sodass der jeweilige Qualitätsmarker zur jeweiligen Genomposition in der Komprimierungseinheit vorliegt,
- Anwenden eines Bewertungsmodells zum Bestimmen eines Vertrauenswertes für den jeweiligen Qualitätsmarker zur jeweiligen Genomposition, sodass ein Vertrauenswert des Qualitätsmarkers zur jeweiligen Genomposition vorliegt,
- Quantisieren des jeweiligen Qualitätsmarkers zur jeweiligen Genomposition anhand des Vertrauenswertes, wobei eine Quantisierungsintensität abhängig vom Vertrauenswert gewählt ist, sodass ein anhand des Vertrauenswertes quantisierter jeweiliger Qualitätsmarker der jeweiligen Genomposition vorliegt,
- Komprimieren des quantisierten Qualitätsmarkers,
sodass die Qualitätsmarker optimiert komprimiert sind.

Es ist dabei ein Kerngedanke der Erfindung, zunächst eine Bewertung zum Erzielen eines Vertrauenswertes für den jeweiligen Qualitätsmarker durchzuführen, um sodann abhängig vom Vertrauenswert des jeweiligen Qualitätsmarkers eine zielgerichtete und individuelle Quantisierung zu wählen. Insgesamt hat sich gezeigt, dass damit bei vertretbarem Informationsverlusten eine möglichst geringe Datenmenge erreicht ist. Es sei hierzu erwähnt, dass insbesondere die Qualitätsmarker zu einer jeweiligen Genomposition bis zu 80 % einer Datenmenge aus einer Genomsequenz ausmachen können, sodass hier eine angepasste und individuell gewählte Komprimierung einen deutlichen Vorteil in der Reduzierung der Datenmenge erzielen kann.

Folgende Begriffe seien in diesem Zusammenhang erläutert:

Ein "Verfahren zum optimierten Komprimieren von Qualitätsmarkern" dient dem verbesserten Komprimieren von Qualitätsmarkern gegenüber bekannten Verfahren. Es handelt sich dabei um eine Handlungsanweisung, welche beispielsweise von Computern ausgeführt werden kann, um ein "Komprimieren" durchzuführen. Ein solches Komprimieren bezeichnet die Reduktion einer Gesamtdatenmenge gegenüber einer Ausgangsdatenmenge, wobei ein Idealfall ein Komprimieren ohne Informationsverlust ermöglicht und, je nach Komprimierungsalgorithmus jedoch entsprechende Informationsverluste in Kauf genommen werden müssen, um beispielsweise eine Datenmenge zu komprimieren. Es ist dabei mit dem Komprimieren jeweils anzustreben, die Reduktion der Datenmenge möglichst hoch und dabei den Informationsverlust möglichst klein zu halten. Die Güte der Komprimierung kann dabei je nach Anwendungsfall in einer möglichst hohen Datenreduktion, einem möglichst verlustfreien Komprimieren oder einem Kompromiss zwischen diesen Zielgrößen bewertet werden.

"Qualitätsmarker" sind Einzelinformationen, welche insbesondere dazu dienen, die Verlässlichkeit einer Information an einer bestimmten Position eines Datensatzes festzustellen und/oder festzuhalten. In diesem Zusammenhang ist ein "Genom" als zusammenhängende Information einer Erbinformation, beispielsweise eines Lebewesens, zu verstehen. Das Genom wird üblicherweise sogenannt "sequenziert", wobei entsprechende Teilinformationen des Genoms als "Genomsequenz" ermittelt werden. Solche Genomsequenzen werden in der Fachsprache auch als "Read" bezeichnet, wobei entsprechende Genomsequenzen aus unterschiedlichen Positionen des Genoms beginnend ermittelt werden und beispielsweise überlappende Positionen in der Genomsequenz bezeichnen können. Solche Positionen, genannt "Genompositionen", bezeichnen dabei eine jeweilige Adresse innerhalb einer Genomsequenz und/oder innerhalb des Genoms, wobei das Genom in diesem Zusammenhang als mit einzelnen Adressen versehene Kette von Genominformationen zu verstehen ist.

Eine "Genominformation" bezeichnet dabei eine Kennung eines jeweiligen Genoms in Form einer Benennung von Nukleo-Basen, welche branchenüblich beispielsweise mit den Buchstaben A, C, G und T bezeichnet werden, womit die unterschiedlichen Nukleo-Basen Adenin, Cytosin, Guanin und Thymin der DNA bezeichnet sind. Eine solche Genominformation kann dabei sowohl aus DNA als auch aus RNA entnommen sein.

Es sei hierzu erwähnt, dass eine solche Genomsequenz mit Laborgeräten bestimmbar ist, jedoch aufgrund der Ausführung entsprechender Apparaturen fehlerbehaftet sein kann. Beispielsweise kann eine einzelne Nukleo-Base fehlerhaft ausgelesen sein, sodass die erzeugte Abfolge von Nukleo-Basen an der dazugehörigen Position im "Read" fehlerhaft ist. Folglich wird bei mittlerweile üblichen Geräten zum Bestimmen der Genomsequenz ein jeweiliger Qualitätsmarker zu jeder Genomposition eines entsprechenden Reads geliefert, welcher eine Aussage über die Qualität des entsprechenden Wertes für die Genominformationen innerhalb der Genomsequenz bezeichnet und wiedergibt. Beispielsweise wird eine unzuverlässige Genominformation hier mit einem Qualitätsmarker anders bezeichnet als eine äußerst zuverlässige Genominformation. Insbesondere für die Weiterverarbeitung entsprechender Genomsequenzen sind diese Qualitätsmarker von hoher Wichtigkeit.

Es sei weiterhin erwähnt, dass entsprechende Genomsequenzen, sofern diese überlappende Positionen innerhalb des Genoms wiedergeben, parallel versetzt zueinander in entsprechende Reihenfolgen gebracht werden können, sodass ein Abgleich zwischen unterschiedlichen Genominformations-Daten aus unterschiedlichen Genomsequenzen für die jeweilige gemeinsame Genomposition bestimmbar ist. Gleiches gilt für zugehörige Qualitätswerte oder zugehörige Qualitätsmarker.

Der "Qualitätswert" einer Genominformation ist beispielsweise der Zahlenwert des jeweiligen Qualitätsmarkers, welcher in beliebiger Darstellung angegeben werden kann. Beispielsweise kann ein solcher Qualitätswert einen Prozentwert zwischen 0 % und 100 % aufweisen, jedoch auch anders dargestellt sein, beispielsweise in einer Buchstabenkodierung oder einem Zahlenwert einer frei gewählten Skala. Kernaussage des Qualitätswertes ist dabei die Verlässlichkeit der jeweiligen Genominformation.

Ein "Einlesen" des jeweiligen Qualitätsmarkers zur jeweiligen Genomposition bezeichnet das Übertragen von Daten in eine Komprimierungseinheit, wobei die Komprimierungseinheit beispielsweise in einem Computer implementiert und mittels eines Algorithmus umgesetzt ist. Beispielsweise könnte jedoch eine solche Komprimierungseinheit auch eine mechanische Schaltanordnung zum Verarbeiten von Dateninformationen sein, auch wenn dieser Fall in der physischen Umsetzung unwahrscheinlich ist.

Ein "Anwenden eines Bewertungsmodells" beschreibt das Aufbringen eines Bewertungsmodells, also eines Algorithmus zum Bestimmen eines Vertrauenswertes eines jeweiligen Qualitätsmarkers, wobei beispielsweise ein Abgleich zwischen unterschiedlichen Qualitätsmarkern an unterschiedlichen Genompositionen erfolgen kann. Auch können beispielsweise zusätzlich unterschiedliche Genominformationen von unterschiedlichen Genompositionen mit in den Abgleich einbezogen werden. Der daraus erzeugte "Vertrauenswert" ist dabei ein Bewertungsmaßstab für die Zuverlässigkeit der Angabe des Qualitätswertes innerhalb des Qualitätsmarkers. So kann beispielsweise aus dem Bewertungsmodell abgeleitet sein, dass ein Vertrauenswert für den jeweiligen Qualitätsmarker sehr gering ist, also die Angabe des Qualitätswertes des jeweiligen Qualitätsmarkers ein geringes Vertrauen genießt.

Erfindungsgemäß erfolgt dann ein "Quantisieren" des jeweiligen Qualitätsmarkers anhand des Vertrauenswertes. Dabei wird beispielsweise bei einem hohen Vertrauenswert des jeweiligen Qualitätsmarkers eine vergleichsweise feine Quantisierung gewählt, wobei die Stärke der Quantisierung hier als "Quantisierungsintensität" bezeichnet ist, wobei eine hohe Quantisierungsintensität mit einer groben Quantisierung gleichzusetzen ist und vice versa. Ist ein entsprechender Vertrauenswert sehr gering, so kann beispielsweise eine niedrige Quantisierungsintensität gewählt werden, um unzuverlässige Qualitätsinformationen nicht noch zusätzlich durch das Quantisieren zu verschlechtern. Analog dazu ist, je nach Anwendungsfall, auch eine inverse Anwendung denkbar, in der beispielsweise eine Quantisierungsintensität für Qualitätsmarker mit einem hohen Vertrauenswert eher gering gewählt wird, beispielsweise um einen hohen Informationsgehalt nicht durch die Komprimierung zu verzerren.

Ein anschließendes "Komprimieren" bezeichnet sodann das Verringern einer Datenmenge der Qualitätsmarker, beispielsweise zum Speichern der Datenmenge mit geringerem Speicherbedarf.

Im Ergebnis liegen dann nach dem Komprimieren der quantisierten Qualitätsmarker "komprimierte Qualitätsmarker" vor, also Dateninformationen bezüglich der Qualitätsmarker mit geringerem Datenumfang. Es sei hierzu erwähnt, dass beim Quantisieren und Komprimieren abhängig vom Vertrauenswert auch unterschiedliche Datenverlustraten akzeptiert werden können.

Um eine zuverlässige Ermittlung eines Vertrauenswertes durchführen zu können, ist das Bewertungsmodell ein Genotyp-Übereinstimmungs-Modell, wobei insbesondere ein Unsicherheitsprofil zu einer Abfolge von Genompositionen ermittelt wird. Ein solches Unsicherheitsprofil zeigt dabei entsprechende Unsicherheitswerte, also eine entsprechende inverse Aussage zum Vertrauenswert, auf.

Alternativ und/oder ergänzend ist das Bewertungsmodell gemäß einer Ausführungsform ein aktivitätsbasiertes nachgeführtes Modell, wobei insbesondere ein Aktivitätsprofil zu einer Abfolge von Genompositionen ermittelt wird.

Ein "Genotyp-Übereinstimmungs-Modell" bestimmt dabei die Wahrscheinlichkeitsverteilung aller theoretisch möglichen Genotypen für jede Genomposition. Dazu wird ein statistisches Modell genutzt. Im Ergebnis wird ein Unsicherheitsprofil bestimmt, welches zu jeweiligen Genompositionen die Unsicherheit der jeweiligen Genominformationen bestimmt. Damit wird die Wahrscheinlichkeit angegeben, mit der eine jeweilige Genominformation korrekt ist. Abhängig von dieser Wahrscheinlichkeit kann dann die Quantisierungsintensität des Qualitätsmarkers einer jeweiligen Genomposition gewählt werden. Dabei kann die Unsicherheit einer Genomposition als eine Metrik betrachtet werden, welche die Wahrscheinlichkeit misst, dass ein bestimmter Genotyp auch der biologisch tatsächlich zugrundeliegende Genotyp ist. Daher dann die Unsicherheit verwendet werden, um die Höhe der Datenverlustrate zu bestimmen, die für alle Qualitätsmarker an einer Genomposition akzeptabel ist.

Ein "aktivitätsbasiertes nachgeführtes Modell", welches alternativ zum vorig genannten Genotyp-Übereinstimmung-Modell angewendet werden kann, nutzt systematische Annahmen, um ein Aktivitätsprofil über die jeweiligen Positionen zu bestimmen. Zu diesen systematischen Annahmen zählt ein verbessertes Modell zur Abschätzung der Wahrscheinlichkeit, dass einige oder mehrere jeweilige Nukleo-Basen fehlerbehaftet sind. Außerdem zählt zu den systematischen Annahmen, dass Genomsequenzen, von denen einige Geninformationen nicht zu Genompositionen zugeordnet werden können, zur Erhöhung von Unsicherheiten in ihrer Genomposition-Umgebung beitragen.

Insbesondere kann das Ermitteln des Vertrauenswertes des jeweiligen Qualitätsmarkers anhand eines insbesondere gewichteten Zuordnens des jeweiligen Qualitätsmarkers zu einem jeweiligen Vertrauensbereich erfolgen. Dabei wird beispielsweise eine Menge von möglichen Vertrauenswerten, beispielsweise zwischen 0 % und 100 %, in entsprechende Intervalle oder Abschnitte gegliedert, wobei ein Zuordnen des jeweiligen Vertrauenswertes zu einem jeweiligen Intervallabschnitt, insbesondere auch gewichtet, erfolgt. So können beispielsweise im Bereich eines hohen Vertrauenswertes zwischen beispielsweise 100 % und 65 % jeweilige Qualitätsmarker ähnlich behandelt werden, wohingegen bei Erreichen deutlich geringerer Vertrauenswerte, beispielsweise unterhalb 65 %, eine feinere Schachtelung erfolgt, um entsprechende Abweichungen besser erfassen zu können. Die Angaben der jeweiligen %-Werte sind hier beispielhaft und können je nach Anwendung variieren.

Gemäß einer Ausführungsform erfolgt das Komprimieren mittels eines Entropiekodierens. Beispielsweise kann das Komprimieren auch mittels eines insbesondere weiteren Quantisierens der Qualitätsmarker erfolgen.

Ein "Entropiekodieren" ist ein Vorgehen zur insbesondere verlustfreien Datenkompression, wobei eine aus einzelnen Zeichen bestehende Zeichenfolge in eine Bitfolge umwandelt wird. Beispielsweise wird dazu eine Huffman-Kodierung oder eine sogenannten arithmetische Kodierung genutzt. Alternativ zu einer Bitfolge kann hier auch eine Folge von Symbolen aus einem Symbolvorrat genutzt werden.

Gemäß einer Ausführungsform erfolgt zum Bestimmen des Vertrauenswertes ein Vergleich mehrerer Genomsequenzen, wobei der Vergleich der Genomsequenzen mittels eines Angleichens von jeweiligen Genompositionen derart erfolgt, dass die jeweiligen Genomsequenzen an jeweiligen angeglichenen Genompositionen jeweilige gleiche Genompositionen der Genomsequenz darstellen. Somit können Genomsequenzen derart zueinander angeordnet werden, dass entsprechende gleiche Genompositionen in unterschiedlichen Genomsequenzen gleichsam parallelisiert sind. Hierbei kann beispielsweise ein Vergleich zwischen entsprechenden Genominformationen an jeweiligen Genompositionen erfolgen, bis beispielsweise eine möglichst hohe Übereinstimmung erfolgt. Die Genomsequenzen werden dabei gleichsam positionsgleich "übereinander gelegt", sodass ein direkter Vergleich der Genominformationen sowie auch der Qualitätsmarker, insbesondere an der jeweiligen Position, erfolgen kann. Insbesondere können sodann jeweilige Qualitätsmarker miteinander abgeglichen werden, sodass ein optimiertes Komprimieren erfolgen kann.

Um eine Datenmenge der komprimierten Qualitätsmarker weiter herabzusetzen, erfolgt zum Speichern der komprimierten Qualitätsmarker ein Versatz, wobei mittels des Versatzes die jeweiligen komprimierten Qualitätsmarker innerhalb einer Zeichentabelle derart versetzt werden, dass eine einstellige Darstellung ermöglicht ist, insbesondere in einem ASCII-Zeichensatz.

Diese Ausführungsform dient insbesondere dazu, beispielsweise mehrstellige ASCII-Zeichen zur Darstellung von Qualitätsmarkern zu vermeiden und sämtliche möglichen Qualitätsmarker in einem Bereich des ASCII-Zeichensatzes zu versetzen, welche einstellig dargestellt werden kann.

Gemäß einer Ausführungsform kann weiterhin ein Glätten der komprimierten Qualitätsmarker und/oder ein Glätten des Unsicherheitsprofils, insbesondere mittels eines Gauß-Filters, erfolgen.

In einem weiteren Aspekt wird die Aufgabe gelöst durch eine Computereinrichtung mit einer Komprimierungseinheit, welche eingerichtet ist, ein Verfahren gemäß einem der vorherigen Ansprüche durchzuführen.

Eine solche "Computereinrichtung" ist dabei insbesondere eine Einrichtung zur elektronischen Datenverarbeitung, welche beispielsweise mittels eines Algorithmus dazu eingerichtet ist, das erfindungsgemäße Verfahren gemäß einer oder mehrerer der obig bezeichneten Ausführungsformen auszuführen. Es kann sich dabei beispielsweise um einen Personal Computer, ein Rechenzentrum, einen Server oder ein vergleichbares informationstechnisches Gerät handeln.

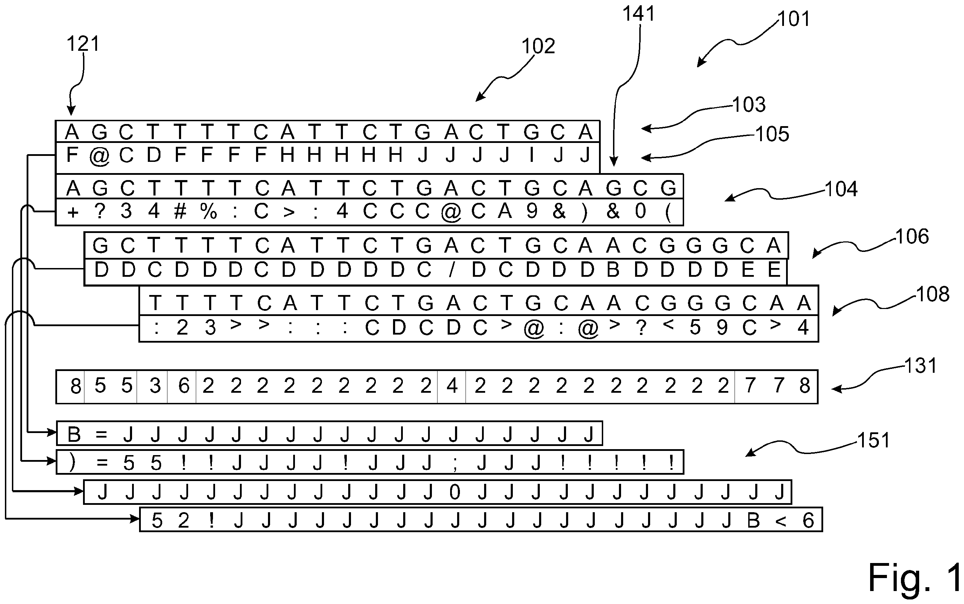

Im Weiteren wird die Erfindung anhand von Ausführungsbeispielen näher erläutert. Es zeigen
- Figur 1: eine Datenmatrix zum Darstellen von Genomsequenzen mit entsprechenden Qualitätsmarkern und daraus abgeleiteten Vertrauenswerten.

Eine Datenmatrix 101 zeigt beispielhaft vier unterschiedliche Genomsequenzen, welche als Genomsequenz 102, 104, 106 und 108 versetzt übereinander matrixartig angeordnet sind. Dabei weist jede Genomsequenz eine Genomposition 121 auf, welche aufsteigend, beispielsweise durchnummeriert, eine Position einer jeweiligen Genominformation 103 im der Genomsequenz zugrundeliegenden Genom darstellt. Jeweils zugeordnet an der gleichen Genomposition 121 weist jede Genominformation 103 einen Qualitätsmarker 105 auf. Der Qualitätsmarker 105 ist Resultat einer Analyse der Genomsequenz während deren Sequenzierung und gibt eine Auskunft darüber, welche Qualität die entsprechende Genominformation aufweist. Gleichsam wird mit dem Qualitätsmarker eine Verlässlichkeit der Information als Zahlenwert, hier als ASCII-Zeichen dargestellt, angegeben. Beispielsweise zeigt ein hoher Qualitätsmarker eine hohe Verlässlichkeit der ausgelesenen Genominformation 105 an der jeweiligen Genomposition 121 auf.

Die Genomsequenzen 102, 104, 106 und 108 resultieren dabei als vier sogenannte unterschiedliche "Reads", also einer mehrfachen nacheinander erfolgten Sequenzierung desselben Genoms.

Es sei hierzu erwähnt, dass eine solche Genomsequenz keinesfalls fehlerfrei ausgelesen werden kann, sondern beispielsweise an einer Genomposition 141 gezeigt in unterschiedlichen Reads an der identischen Genomposition 141 unterschiedliche Werte annehmen kann (hier "G", "A"). Hinzu ist zu beachten, dass auch der Qualitätsmarker 105 jeweils unterschiedlich ausfällt. Insgesamt besteht also eine Unsicherheit, welche Genominformation an der Position 141 tatsächlich im zugrundeliegenden Genom vorliegt.

Insbesondere die Qualitätsmarker 105 weisen dabei eine hohe Datenmenge auf, sodass deren Komprimierung wünschenswert ist.

Es erfolgt nun eine Analyse der Qualitätsmarker 105 zum Festlegen eines Vertrauenswertes. Dazu wird der Qualitätsmarker einem Bewertungsmodell, im gezeigten Beispiel einem Genotyp-Übereinstimmungs-Modell unterzogen, sodass aus diesem Bewertungsmodell heraus Vertrauenswerte 131 für jeweilige Positionen erzeugt sind. Unterschiedliche Vertrauenswerte zeigen dabei über eine Anzahl von Genomsequenzen die Verlässlichkeit der jeweiligen Qualitätsmarker 105 im Querschnitt.

Ein "Genotyp-Übereinstimmungs-Modell" bestimmt dabei beispielhaft die Wahrscheinlichkeitsverteilung aller theoretisch möglichen Genotypen für Genomposition 141. Unter der Annahme, dass das zugrundeliegende Genom human und damit diploid ist, ergeben sich 10 mögliche Genotypen, welche sich durch zweifaches Ziehen, also Auslesen, mit Wiederholung ohne Beachtung der Reihenfolge aus der Menge an möglichen Nukleo-Basen ergeben: AA, AC, AG, AT, CC, CG, CT, GG, GT, TT. Betrachtet man die an Genomposition 141 vorliegenden Genominformationen (G, A, A) mit den zugehörigen Qualitätsmarkern ("&", "B", ">"), welche eine Qualitätsangabe in aufsteigender Reihenfolge gemäß"schlecht", "mittelgut", "mittelschlecht" ausdrücken, so wird eine Wahrscheinlichkeitsverteilung erhalten, in welcher mehrere Genotypen wahrscheinlich erscheinen, z. B. AA und AG, aber auch AC und AT, aufgrund der qualitativ "schlecht" bewerteten Genominformation G. Im Ergebnis ist die Unsicherheit an Genomposition 141 also vergleichsweise hoch.

Sodann erfolgt eine Quantisierung der Qualitätsmarker 105 anhand der ermittelten Vertrauenswerte jeweils für eine jeweilige Genomposition 121. Im Ergebnis werden dann quantisierte Qualitätsmarker 151 erzeugt, welche (vergleiche Figur 1) eine deutlich höhere Gleichförmigkeit aufweisen als die ursprünglichen Qualitätsmarker 105 in der Datenmatrix 101.

Die quantisierten Qualitätsmarker werden dann einer Komprimierung, beispielsweise einer Entropiecodierung, unterzogen.

Im Ergebnis liegen dann komprimierte gewichtete Qualitätsmarker vor.

### Bezugszeichenliste

- 101: Datenmatrix
- 102: Genomsequenz
- 103: Genominformation
- 104: Genomsequenz
- 105: Qualitätsmarker
- 106: Genomsequenz
- 108: Genomsequenz
- 121: Genomposition
- 131: Vertrauenswert
- 141: Genomposition
- 151: gewichtete Qualitätsmarker

## Patentansprüche

1. Verfahren zum optimierten Komprimieren von Qualitätsmarkern (105) einer aus einem Genom bestimmten Genomsequenz (102, 104, 106, 108) mittels einer Komprimierungseinheit, wobei die Genomsequenz (102, 104, 106, 108) an jeweiligen Genompositionen (121) jeweilige Genominformationen (103) aufweist und jeweilige Qualitätsmarker (105) der jeweiligen Genomposition (121) zugeordnet sind und einen Qualitätswert einer Genominformation (103) an der jeweiligen Genomposition (121) in der Genomsequenz (102) aufweisen, mit folgenden Schritten:
- Einlesen des jeweiligen Qualitätsmarkers (105) zur jeweiligen Genomposition (121, 141) in die Komprimierungseinheit, sodass der jeweilige Qualitätsmarker (105) zur jeweiligen Genomposition (121, 141) in der Komprimierungseinheit vorliegt,
- Anwenden eines Bewertungsmodells zum Bestimmen eines Vertrauenswertes für den jeweiligen Qualitätsmarker (105) zur jeweiligen Genomposition (121, 141), sodass ein Vertrauenswert des Qualitätsmarkers (105) zur jeweiligen Genomposition (121, 141) vorliegt,
- Quantisieren des jeweiligen Qualitätsmarkers zur jeweiligen Genomposition (121, 141) anhand des Vertrauenswertes, wobei eine Quantisierungsintensität abhängig vom Vertrauenswert gewählt ist, sodass ein anhand des Vertrauenswertes quantisierter jeweiliger Qualitätsmarker (105) der jeweiligen Genomposition (121, 141) vorliegt,
- Komprimieren des jeweiligen quantisierten Qualitätsmarkers,
sodass die Qualitätsmarker optimiert komprimiert sind.

2. Verfahren gemäß Anspruch 1, **dadurch gekennzeichnet, dass** das Bewertungsmodell ein Genotyp-Übereinstimmungs-Modell ist, wobei insbesondere ein Unsicherheitsprofil zu einer Abfolge von Genompositionen (121, 141) ermittelt wird.

3. Verfahren gemäß Anspruch 1 oder 2, **dadurch gekennzeichnet, dass** das Bewertungsmodell ein aktivitätsbasiertes nachgeführtes Modell ist, wobei insbesondere ein Aktivitätsprofil zu einer Abfolge von Genompositionen (121, 141) ermittelt wird.

4. Verfahren gemäß einem der vorherigen Ansprüche, **dadurch gekennzeichnet, dass** das Ermitteln des Vertrauenswertes des jeweiligen Qualitätsmarkers (105) anhand eines insbesondere gewichteten Zuordnens des jeweiligen Qualitätsmarkers (105) zu einem jeweiligen Vertrauensbereich, erfolgt.

5. Verfahren gemäß einem der vorherigen Ansprüche, **dadurch gekennzeichnet, dass** das Komprimieren mittels eines Quantisierens der Qualitätsmarker (105), insbesondere mit einer nachfolgenden Entropiecodierung, erfolgt.

6. Verfahren gemäß einem der vorherigen Ansprüche, **dadurch gekennzeichnet, dass** zum Bestimmen des Vertrauenswertes ein Vergleich mehrerer Genomsequenzen (102, 104, 106, 108) erfolgt, wobei der Vergleich der Genomsequenzen (102, 104, 106, 108) mittels eines Angleichens von jeweiligen Genompositionen (121, 141) derart erfolgt, dass die jeweiligen Genomsequenzen (102, 104, 106, 108) an jeweiligen angeglichenen Genompositionen (121, 141) jeweilige gleiche Genompositionen (121, 141) der Genomsequenz (102, 104, 106, 108) darstellen.

7. Computereinrichtung mit einer Komprimierungseinheit, welche eingerichtet ist, ein Verfahren gemäß einem der vorherigen Ansprüche durchzuführen.
